# EUROPÄISCHE PATENTSCHRIFT

(11) **EP 2 654 548 B1**
(45) Veröffentlichungstag und Bekanntmachung des Hinweises auf die Patenterteilung: **20.04.2016**
(21) Anmeldenummer: 12703077.3
(22) Anmeldetag: 03.02.2012
(51) Int. Cl.: A61B 1/04, A61B 1/00, A61B 5/07, A61B 5/06, G06T 7/00, G06T 7/40

(54) **VERFAHREN UND EINRICHTUNG ZUR UNTERSUCHUNG EINES HOHLORGANS MIT EINER MAGNETGEFÜHRTEN ENDOSKOPKAPSEL**
METHOD AND DEVICE FOR EXAMINING A HOLLOW ORGAN USING A MAGNET-GUIDED ENDOSCOPE CAPSULE
PROCÉDÉ ET DISPOSITIF SERVANT À EXAMINER UN ORGANE CREUX AU MOYEN D'UNE CAPSULE ENDOSCOPIQUE GUIDÉE PAR MAGNÉTISME

(30) Priorität: 15.02.2011 DE 102011004160
(43) Veröffentlichungstag der Anmeldung: 30.10.2013
(73) Patentinhaber: Given Imaging Ltd., 20692 Yoqneam (IL)
(72) Erfinder: MEWES, Philip, 90439 Nürnberg (DE); NEUMANN, Dominik, 92245 Kümmersbruck (DE); FÖRTSCH, Stefan, 91358 Kunreuth (DE); JULOSKI, Aleksandar, 90453 Nürnberg (DE); KELLER, Henrik, 91054 Erlangen (DE)
(74) Vertreter: HGF Limited
(86) Internationale Anmeldenummer: PCT/EP2012/051821
(87) Internationale Veröffentlichungsnummer: WO 2012/110324

(56) Entgegenhaltungen:
- EP-A1- 2 033 567
- WO-A2-2011/005865
- GB-A- 2 466 818
- US-A- 5 220 614
- US-A1- 2003 053 660
- US-A1- 2003 077 223
- US-A1- 2003 095 721
- US-A1- 2004 052 418
- US-A1- 2010 054 595

## Beschreibung

Einrichtung zur Untersuchung eines Hohlorgans mit einer magnetgeführten Endoskopkapsel

Die Erfindung bezieht sich auf eine Einrichtung zur Untersuchung eines Hohlorgans mit einer magnetgeführten Endoskopkapsel.

Zur optischen Untersuchung der Innenoberfläche eines im Körper eines Lebewesens befindlichen Hohlorgans, beispielsweise des Magen-Darmtraktes eines Patienten, ist es beispielsweise aus der DE 101 42 253 C1 bekannt, in das Hohlorgan eine Endoskopkapsel, bei der Untersuchung des Magen-Darmtraktes beispielsweise durch Schlucken einzubringen, die ohne feste Verbindung nach außen im Hohlorgan durch externe Magnetfelder frei manövrierbar ist.

Bei einer solchen endoskopischen Untersuchung (magnetgeführte Kapselendoskopie) muss der die Diagnose durchführende Arzt eine Vielzahl von in kurzen Zeitabständen aufeinanderfolgenden Bildern einerseits hinsichtlich gegebenenfalls vorhandener diagnostisch relevanter Bildinhalte, andererseits auch im Hinblick auf eine korrekte Steuerung der Endoskopkapsel verarbeiten. Allein die Steuerung der Endoskopkapsel erfordert jedoch ein hohes Maß an Aufmerksamkeit, so dass grundsätzlich das Risiko besteht, dass der die Untersuchung durchführende Arzt diagnostisch relevante Details übersieht.

Zwar ist es bei der passiven Kapselendoskopie grundsätzlich bekannt, das vorhandene Bildmaterial nachträglich einer digitalen Bildverarbeitung zu unterziehen, um auf diese Weise mit Methoden der computerunterstützten Diagnose (CAD) Verfahren zur Verfügung zu stellen, mit denen es möglich ist, klinisch relevante pathologische Strukturen automatisch zu erkennen und auf diese Weise dem untersuchenden Arzt die Auswertung zu erleichtern.

Eine solche nachträgliche automatisierte Auswertung würde jedoch auch bei der magnetgeführten Kapselendoskopie auf der Grundlage des verfügbaren Videomaterials erfolgen, so dass nach wie vor das Risiko besteht, dass pathologische Erscheinungsformen nicht oder nur undeutlich auf den Videobildern wiedergegeben werden, da diese von der Endoskopkapsel nicht oder nur undeutlich erfasst worden sind, weil der untersuchende Arzt die diagnostische Relevanz während der Untersuchung nicht erkannt und bei der Steuerung der magnetgeführten Endoskopkapsel nicht entsprechend berücksichtigt hat.

Der Erfindung liegt daher die Aufgabe zugrunde, eine Einrichtung zur Untersuchung eines Hohlorgans mit einer magnetgeführten Endoskopkapsel anzugeben, mit dem die Wahrscheinlichkeit, mit der pathologische Strukturen in Hohlorgan erkannt werden können, erhöht und dementsprechend die Wahrscheinlichkeit einer falsch-negativen oder falsch-positiven Diagnose verringert ist.

Die genannte Aufgabe wird gemäß der Erfindung gelöst, mit einer Einrichtung mit den Merkmalen des Patentanspruches 1. Gemäß diesen Merkmalen werden die aus dem Inneren des Hohlorgans fortlaufend mit der magnetgeführten Endoskopkapsel erzeugten Videobilder während der Navigation der Endoskopkapsel im Hohlraum mit Methoden der digitalen Bildverarbeitung automatisch auf das Vorhandensein diagnostisch relevanter Information analysiert, und es wird das Ergebnis der Analyse, d.h. eine Aussage darüber, ob die Videobilder eine diagnostisch relevante, d.h. auf ein Krankheitsbild hinweisende Information enthalten oder nicht, zur Steuerung der Endoskopkapsel herangezogen. Die Analyse umfasst dabei die folgenden weiteren Schritte:
a) aus den Videobildern wird zum Lokalisieren von hohen Intensitätsgradienten durch Vorbearbeitung jeweils ein Grauwertbild erzeugt, dessen Bildpunkten jeweils ein Grauwert zugeordnet wird,
b) aus dem Grauwertbild wird durch Glättung und Kantenfilterung ein gefiltertes Bild erzeugt, dessen Bildpunkten ein Filterwert zugeordnet ist,
c) aus den Filterwerten der Bildpunkte des gefilterten Bildes wird ein Schwellwert abgeleitet,
d) das Videobild wird in eine Vielzahl von disjunkten Gruppen aufgeteilt, die jeweils eine Vielzahl von Bildpunkten umfassen,
e) aus diesen Gruppen werden als relevante Gruppen diejenigen Gruppen ausgewählt, die im gefilterten Bild eine Mindestanzahl von Bildpunkten enthalten, die den Schwellwert überschreiten,
f) jeder relevanten Gruppe wird eine Vielzahl von Eigenschaften zugeordnet, anhand derer mit einem mit einer Vielzahl von bewerteten Testbildern trainierten Expertensystem während der Navigation automatisch analysiert wird, ob die relevante Gruppe einen diagnostisch relevanten Bereich indiziert oder nicht.

Durch diese Maßnahmen wird die Anzahl der zur Analyse benötigten Bildpunkte auf ein Maß reduziert, das eine automatisierte Analyse während der Untersuchung (on-the-fly) ermöglicht.

Die auf ein Krankheitsbild hinweisende, d.h. diagnostisch relevante Information kann beispielsweise direkt aus einer im Videobild erkennbaren pathologischen Veränderung an der Wand des Hohlorgans oder indirekt aus den Opazitätseigenschaften der im Hohlorgan befindlichen Flüssigkeit abgeleitet werden, da sich bestimmte Krankheitsbilder auch durch lokal variierende Trübung oder Färbung der Flüssigkeit oder durch das Vorhandensein von Partikeln in der Flüssigkeit bemerkbar machen. Die Einrichtung ist besonders geeignet zum Erkennen einer akuten Gastritis (Magenschleimhautentzündung). Das Erkennen einer solchen Gastritis ist von besonderem Interesse, da diese eine der Hauptursachen für das Auftreten von Magenkrebs ist. Vorzugsweise besteht deshalb die diagnostische relevante Information in dem Vorhandensein von Bereichen an der Wand des Hohlorgans, die eine mögliche Gastritis indizieren.

Die Erfindung beruht dabei auf der Überlegung, dass das Erkennen diagnostisch relevanter Bereiche der Wand oder diagnostisch relevanter Eigenschaften der Flüssigkeit, in der die Endoskopkapsel schwimmt, während der Untersuchung dazu benutzt werden kann, die Endoskopkapsel, d. h. deren Bewegung und Ausrichtung innerhalb des Hohlorgans zu steuern, um den als möglicherweise pathologisch identifizierten Wandbereich des Hohlorgans oder die in der Flüssigkeit selbst entdeckten Unregelmäßigkeiten einer ausführlicheren endoskopischen Untersuchung zu unterziehen, indem beispielsweise die Endoskopkapsel angehalten wird und näher an den betreffenden Oberflächenbereich herangefahren wird, um dem Arzt eine genauere Betrachtung zu ermöglichen. Dies kann beispielsweise eine höhere Vergrößerung oder eine Betrachtung aus unterschiedlichen Winkeln sein, die ihm eine bessere Beurteilung ermöglicht, ob es sich tatsächlich um einen gastritischen Bereich handelt oder nicht. Beim Erkennen einer diagnostisch relevanten Veränderung der Flüssigkeit kann beispielsweise die Endoskopkapsel gedreht werden, um die Eigenschaften der Flüssigkeit in unterschiedlichen Richtungen näher zu analysieren, um beispielsweise die Ursache der Veränderung, beispielsweise der Ort an der Wand, an dem eine Ablösung von Partikeln stattfindet, lokalisieren zu können, so dass dieser näher untersucht werden kann.

Diese Steuerung kann entweder automatisiert erfolgen oder vom Arzt manuell durchgeführt werden, wenn ihm bei der Betrachtung der Videobilder am Monitor optisch oder visuell ein derartiger Hinweis auf das mögliche Vorhandensein einer pathologischen Veränderung angezeigt wird.

Ein Verfahren mit den vorstehend genannten Verfahrensschritten a) bis f) eignet sich insbesondere zum Erzeugen der für die automatisierte Analyse erforderlichen bewerteten Testbilder, indem die mit diesen Verfahrensschritten aus einem Videobild erzeugten relevanten Gruppen manuell als diagnostisch relevant oder diagnostisch irrelevant klassifiziert, d. h. von einem menschlichen Experten betrachtet und bewertet werden.

Zur weiteren Erläuterung der Erfindung wird auf die Figuren verwiesen. Es zeigen:
Fig. 1 eine Einrichtung gemäß der Erfindung in einer schematischen Prinzipdarstellung,
Fig. 2a,b ein Flussdiagramm, in dem der Ablauf der automatischen Analyse der von der Endoskopkapsel bereitgestellten Videobilder dargestellt ist,
Figuren 3 und 4 jeweils ebenfalls schematisch vereinfacht Bilder vom Inneren eines Magens mit unterschiedlichen pathologischen Erscheinungsformen innerhalb einer im Magen befindlichen Flüssigkeit.

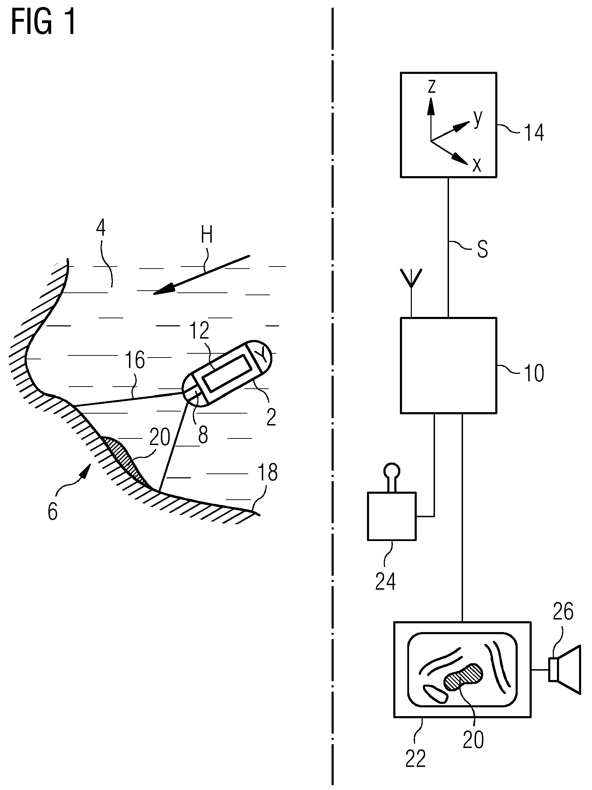

Gemäß Figur 1 befindet sich eine magnetgeführte Endoskopkapsel 2 in einem mit einer Flüssigkeit 4 gefüllten Hohlorgan 6, beispielsweise einem Magen. Mit zumindest einer in der Endoskopkapsel 2 angeordneten Videokamera 8 werden fortlaufend Videobilder aufgenommen und über Funk an eine externe Steuer- und Auswerteeinrichtung 10 übermittelt. In der Endoskopkapsel 2 ist ein Permanentmagnet 12 angeordnet. Die Führung, d.h. die Bewegung und Ausrichtung der Endoskopkapsel 2 erfolgt mit Hilfe eines inhomogenen Magnetfeldes H, das von einem externen Magnetsystem 14 erzeugt wird, das beispielsweise drei Magnetspulen umfasst, deren Spulenachsen senkrecht zueinander orientiert sind und ein Gerätebezugssystem xyz festlegen.

Im dargestellten Beispiel befindet sich im Gesichtsfeld 16 der Videokamera an der Wand 18 des Hohlorgans eine pathologische Struktur 20, beispielsweise eine lokale Entzündung (gastritischer Bereich). Die von der Videokamera 8 aufgenommenen und an die Steuer- und Auswerteeinrichtung 10 übermittelten Videobilder werden auf einem Monitor 22 wiedergegeben. Die pathologische Struktur 20 ist auf dem Monitor 22 zu erkennen und durch Schraffur hervorgehoben.

Das Magnetsystem 14 wird mit Hilfe von Steuersignalen S gesteuert, die von der Steuer- und Auswerteeinrichtung 10 entweder automatisch oder durch Eingaben eines Benutzers über eine externe Eingabeeinheit 24, im Beispiel veranschaulicht durch einen Joystick, generiert werden.

In der Steuer- und Auswerteeinheit 10 sind Bildverarbeitungsalgorithmen implementiert, mit denen es möglich ist, pathologische Strukturen 20 automatisch zu erkennen und im Videobild zu markieren, wie dies durch die Schraffur angedeutet ist. Darüber hinaus können noch weitere optische oder akustische Anzeigen, in der Fig. 1 durch einen Lautsprecher 26 symbolisiert, vorgesehen sein, die den Bediener auf das Vorhandensein einer von der Steuer- und Auswerteeinheit 10 als pathologisch klassifizierten Struktur 20 hinweisen, um diesen die Möglichkeit zu verschaffen, diese möglicherweise pathologische Struktur 20 näher zu untersuchen, indem er beispielsweise die Endoskopkapsel 2 anhält oder näher an die Struktur 20 heranfährt, um sicherer beurteilen zu können, ob es sich tatsächlich um eine pathologische Erscheinung handelt oder nicht.

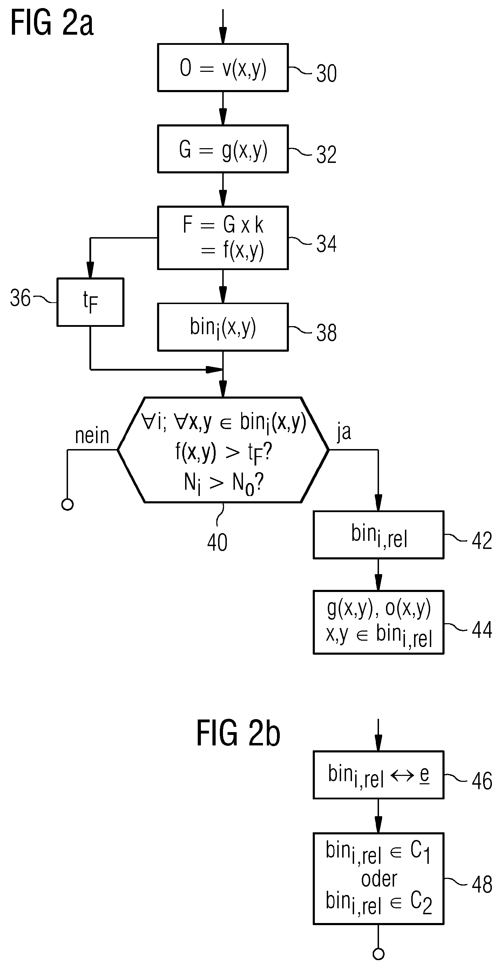

Die in der Steuer- und Auswerteeinheit 10 erfolgende Bildverarbeitung und Bildauswertung ist in Figuren 2a und b anhand eines Flussdiagrammes veranschaulicht. In einem ersten Schritt 30 wird das originale farbige Videobild O = v(x,y) gespeichert, wobei jedem Bildpunkt x,y ein dreikanaliger RGB-Wert v zugeordnet ist. Für die weitere Verarbeitung wird nun in einem nächsten Schritt 32 aus diesem Videobild O = v(x,y) ein Grauwertbild G = g(x,y) berechnet, wobei jedem Bildpunkt x,y ein Grauwert g auf einer beispielsweise 256 Grauwerte umfassenden Skala zugeordnet ist. Grundlage hierfür ist die Überlegung, dass im Übergangsbereich zwischen einer pathologischen Struktur, beispielsweise ein gastritischer Bereich, und der an diese angrenzenden nicht-pathologischen Struktur, beispielsweise ein nicht-gastritischer Bereich ausgeprägte Intensitätswechsel vorliegen, die sich auch im einkanaligen Grauwertbild G wiederfinden. In diesem Grauwertbild G können dann hohe Intensitätsgradienten mit 2D-Filtern beispielsweise nach vorhergehender Glättung mit einem Kantenfilter lokalisiert werden. Das Grauwertbild G wird dementsprechend in Schritt 34 mit einem Filterkern K gefaltet, der vorzugsweise selbst eine Faltung aus einem Gauß-Filter und einem Laplacefilter ist (ein sogenannter L(aplacian)o(f)G(Gaussian)-Filter), um Regionen mit stark ausgeprägten Kanten, an denen sich die Intensität mit hoher Ortsfrequenz ändert, hervorzuheben. Auf diese Weise entsteht ein gefiltertes Bild F = GxK = f(x,y), bei dem jedem Bildpunkt x,y ein Filterwert f zugeordnet ist. Die Anzahl der Spalten bzw. Reihen des verwendeten Filterkerns entspricht dabei in etwa der Anzahl der Bildpunkte (Pixel), die sich in einer die Kante bildenden Übergangszone zwischen einem entzündeten Bereich und einem gesunden Bereich befinden. In der Praxis hat sich dabei ein Filter mit 9x9=81 Matrixelementen mit einer Varianz σ = 1,4 bewährt.

In diesem gefilterten Bild F = f(x,y) wird nun im Schritt 36 nach einem ersten Schwellwert t_{F} gesucht, mit dessen Hilfe relevante Amplitudenänderungen von nicht relevanten Amplitudenänderungen getrennt werden können. Da sich die erzeugten Videobilder hinsichtlich ihres Kontrastes und der Beleuchtungsbedingungen erheblich voneinander unterscheiden, ist hierzu ein fest vorgegebener Schwellwert ungeeignet. Zur Bestimmung des ersten Schwellwertes t_{F} wird deshalb für jedes gefilterte Bild F = f(x,y) ein Histogramm mit beispielsweise 256 gleichgroßen Intervallen gebildet, dessen untere Grenze durch den minimalen Filterwert und dessen oberen Grenze durch den maximalen Filterwert gebildet ist. Hierzu wird im Histogramm analysiert, in welchem Intervall ein Viertel des maximalen Filterwertes unterschritten wird. Die untere Grenze dieses Intervalls legt den ersten Schwellwert fest. Mit dem auf diese Weise festgelegten Schwellwert t_{F} wird eine Maske oder ein Binärbild B = B(x,y) gebildet, in dem denjenigen Bildpunkten x,y der Wert b = 1 zugeordnet wird, deren Filterwert f im gefilterten Bild F = f(x,y) größer als der Schwellwert t_{F} ist. Den übrigen Bildpunkten x,y wird der Wert b = 0 zugeordnet.

In einem nächsten Schritt 38 wird nun das Binärbild B = b(x,y) in eine Mehrzahl i=1,...,n von disjunkten Gruppen (Bins) binᵢ(x,y) aufgeteilt, die jeweils eine Mehrzahl von Bildpunkten x,y umfassen, wobei die Gesamtheit der Gruppen binᵢ(x,y) das gesamte Bild abdecken. Im Beispiel werden Gruppen binᵢ(x,y) aus jeweils 15x15 Bildpunkten gebildet. Diese Gruppen binᵢ(x,y) werden nun in einem nächsten Schritt 40 dahingehend analysiert, wie viele der Bildpunkte x,y des Binärbildes B = b(x,y) innerhalb einer solchen Gruppe binᵢ (x,y) den Wert b = 1 aufweisen, d. h. es wird die Anzahl Nᵢ der innerhalb der Gruppe binᵢ(x,y) vorhandenen Bildpunkte x,y bestimmt, deren Wert b im Binärbild B = b(x,y) gleich 1 ist. Für die weitere Bearbeitung werden nur diejenigen Gruppen binᵢ(x,y) berücksichtigt, in denen die Anzahl Nᵢ solcher Bildpunkte x,y größer als ein Minimalwert N₀ ist, der im Beispiel mit 10 angesetzt ist. Mit anderen Worten: Wenigstens 10 der 225 Bildpunkte x,y einer Gruppe binᵢ(x,y) müssen im Binärbild B = b(x,y) den Wert b = 1 bzw. im gefilterten Bild F = f(x,y) einen Filterwert f aufweisen, der den Schwellwert t_{F} überschreitet.

Diese Vorgehensweise beruht auf der Überlegung, dass sowohl schnelle Intensitätsänderungen im Bild, wie sie beispielsweise bei einer gastritischen Läsion auftreten, als auch hochfrequentes Rauschen zu einer Häufung von Filterantworten mit großen Amplitudenänderungen führen, wobei letztere jedoch nur isoliert und mit kleiner Anzahl auftreten. Auf diese Weise verbleiben gemäß Schritt 42 relevante Gruppen bin_{i,rel} (x,y), die im Originalbild diejenigen Bereiche markieren, die der weiteren Analyse unterzogen werden.

In einem nächsten Schritt 44 wird auf die Original-Bilddaten v(x,y) des Videobildes O dieser relevanten Gruppen bin_{i,rel}(x,y), d.h. die originalen RGB-Werte der einzelnen verbliebenen relevanten Bildpunkte x,y zurückgegriffen. Die Original-Bilddaten v jeder relevanten Gruppe bin_{i,rel}(x,y) werden nun in Schritt 46 hinsichtlich einer Vielzahl von Eigenschaften analysiert. Dies sind im vorliegenden Fall Farbeigenschaften, Eigenschaften des Farbhistogramms, Farbstruktureigenschaften, Textureigenschaften und Filtereigenschaften. Ein Beispiel für Farbeigenschaften ist der Mittelwert und der Maximalwert für jeden Kanal des RGB-Farbraumes innerhalb einer solchen Gruppe bin_{i,rel}(x,y). Zusätzlich werden Farbhistogramme für den R-Kanal im RGB-Farbraum, den dritten Kanal im HSV-Farbraum und dem L-Kanal des Lab-Farbraumes gebildet.

Die Farbstruktureigenschaften dienen dazu, zwischen der quantitativen und räumlichen Verteilung von Farbeigenschaften innerhalb einer Gruppe bin_{i,rel}(x,y) zu unterscheiden. Hierzu werden die Gruppen bin_{i,rel}(x,y) jeweils in Untergruppen unterteilt. Im Ausführungsbeispiel werden in jeder der Gruppen bin_{i,rel}(x,y) 25 Untergruppen zu je 3 x 3 Bildpunkten gebildet. Nunmehr wird in fünf Farbkanälen (RGB-Farbraum: R, G, B; Lab-Farbraum: L; HSV-Farbraum: V) das Auftreten einer speziellen Farbeigenschaft detektiert. Als Merkmal wird dann die Häufigkeit des Auftretens einer bestimmten Farbeigenschaft in einem dieser Kanäle innerhalb der Untergruppe aufgenommen.

Zum Untersuchen der Textureigenschaften innerhalb einer jeden relevanten Gruppe bin_{i,rel}(x,y) wird außerdem auf die in dieser Gruppe vorliegenden Grauwerte g des ungefilterten Grauwertbildes G zurückgegriffen und ein sogenanntes differenceof-boxes-Filter angewandt, dessen Filterantwort hohe negative und positive Amplituden für räumliche Änderungen der Intensität haben.

Auf diese Weise wird jeder relevanten Gruppe bin_{i,rel}(x,y) ein Eigenschaftsvektor e mit einer der Anzahl m der Eigenschaften entsprechenden Anzahl von Komponenten zugeordnet.

In vorhergehenden Lernschritten sind für eine Vielzahl von Testbildern die Original-Bilddaten der in jedem der Testbilder nach dem vorstehend erläuterten Verfahren extrahierten relevanten Gruppen bin_{i,rel}(x,y) von einem Experten hinsichtlich ihrer diagnostischen Relevanz beurteilt. Hierzu werden zwei Klassen C1, C2 gebildet. Relevante Gruppen bin_{i,rel}(x,y) mit positivem Befund (diagnostisch relevant) werden der Klasse C1 zugeordnet, die übrigen (diagnostisch irrelevant) der Klasse C2 (Schritt 48).

Mit Hilfe eines Lernalgorithmus, im Beispiel ein sogenannter Adaptive-Boosting-Algorithmus, wird nun anhand der von Experten beurteilten Testbilder das System derart trainiert, dass dieses auf der Grundlage des jeder relevanten Gruppe bin_{i,rel}(x,y) zugeordneten Eigenschaftsvektors mit einer hohen Wahrscheinlichkeit treffsicher entscheiden kann, welcher Klasse C1, C2 diese Gruppe bin_{i,rel}(x,y) zugeordnet werden kann.

Im Verlauf dieser Trainingsphase werden außerdem die für die spätere automatisierte Bildanalyse verwendeten Eigenschaften, d. h. die tatsächlich notwendigen und geeigneten, d.h. relevanten Komponenten des Eigenschaftsvektors aus einer größeren Anzahl von Eigenschaften extrahiert, indem diejenigen Komponenten nicht mehr berücksichtigt werden, die in der Trainingsphase sowohl für die Gruppen bin_{i,rel}(x,y) der Klasse C1 als auch für die Gruppen bin_{i,rel}(x,y) der Klasse C2 den Wert 0 ergeben. Ebensowenig werden diejenigen Eigenschaften weiter berücksichtigt, deren Varianz in der Gruppe C1 größer oder annähernd gleich der Varianz dieses Merkmals in der Gesamtzahl der Gruppen ist.

Mit diesen Maßnahmen hat sich in der Praxis eine Anzahl von 66 Merkmalen ergeben, die bei der automatisierten Bildauswertung zur Beurteilung herangezogen werden, ob es sich um eine gastritische Läsion handelt oder nicht.

Mit dem auf diese Weise trainierten Expertensystem wird nun in Schritten 46 und 48 beurteilt, welcher Klasse C1, C2 die in einem während einer endoskopischen Untersuchung erzeugten Videobild mit den Schritten 30 bis 42 identifizierten relevanten Gruppen bin_{i,rel}(x,y) zuzuordnen sind. Mit anderen Worten: Das Expertensystem beurteilt automatisch, ob die relevante Gruppe bin_{i,rel} einen gastritischen Bereich indiziert oder nicht. Wenn relevante Gruppen bin_{i,rel} eines Videobildes der Klasse C1 zugeordnet worden sind, wird dies akustisch oder optisch, beispielsweise innerhalb des Videobildes durch eine entsprechende Hervorhebung dem Nutzer angezeigt, so dass dieser aktiv in die Steuerung der Endoskopkapsel eingreifen kann. Ergänzend oder alternativ zu einer solchen manuellen Steuerung kann auch eine automatische Steuerung der Endoskopkapsel vorgesehen sein.

Das vorstehend erläuterte Verfahren ist insbesondere für die Erkennung einer Gastritis geeignet und kann grundsätzlich mit denselben vorverarbeitenden, zur Auswahl der relevanten Gruppen bin_{i,rel}(x,y) führenden Schritten auch zur automatischen Erkennung anderer Krankheitsbilder verwendet werden, bei denen die diagnostisch relevante Information in einer örtlich variablen Trübung und/oder Färbung einer im Hohlorgan befindlichen Flüssigkeit und/oder einem Vorhandensein von Partikeln in der Flüssigkeit besteht, d.h. bei denen die optischen Eigenschaften der Flüssigkeit das Vorliegen eines Krankheitsbildes indizieren.

So vermischt sich bei bestimmten Krankheitsbildern im untersuchten Organ die zugeführte Flüssigkeit, deren Eigenschaften bekannt sind, mit Fremdkörpern. So kann es sich bei diesen Fremdkörpern beispielsweise um Gewebeteile handeln, die sich flockenartig bei einem Ulcus ventriculi (Magengeschwür) von der Magenwand ablösen. Diese Gewebeflocken sind dann im Endoskopbild als in der Flüssigkeit schwebende Partikel zu erkennen. Die Art, Menge und Größe dieser Fremdkörper geben dabei ein sehr gutes erstes Indiz dafür ab, ob eine bestimmte Krankheit vorliegt, auch wenn die betroffene anatomische Stelle mit dem Endoskop noch nicht gesichtet wurde. Zu erkennen sind die Fremdkörper im Endoskop zumeist als schwebende Partikel, können aber bei geringer Größe auch als Eintrübung der Flüssigkeit ausgebildet sein.

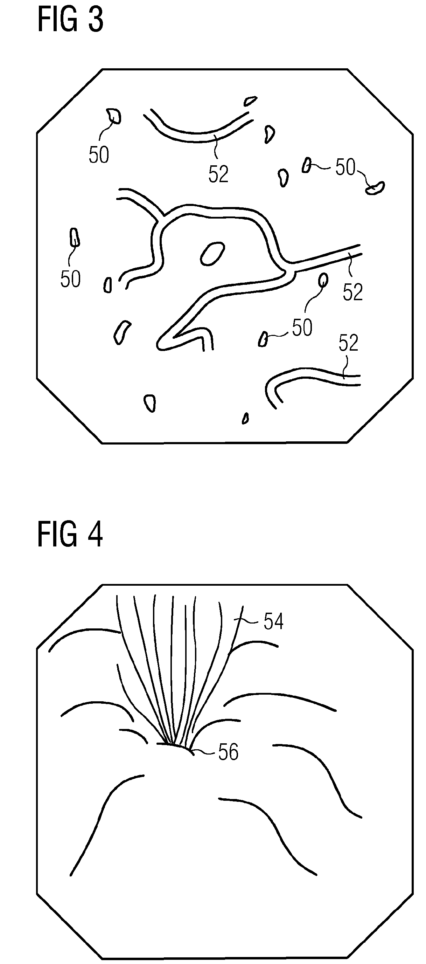

In Figur 3 ist schematisch ein Endoskopbild wiedergegeben, bei dem sich als Folge eines Magengeschwürs Exsudat von der Magenwand gelöst hat und als weißliche flockenartige Fremdkörper 50 in der Flüssigkeit schwimmt. Bei einer automatischen Detektion kann dabei ausgenutzt werden, dass diese Flocken 50 gegenüber den im Hintergrund an der Magenwand befindlichen, ebenso wie die Magenwand rötlichen Strukturen 52 als helle weiße Bildbereiche deutlich erkennbar werden und somit automatisch segmentiert und hinsichtlich Größe und Anzahl analysiert werden können.

In Figur 4 ist ein weiteres Krankheitsbild dargestellt, bei der Galle als gelblich-grüne bis bräunliche Flüssigkeit über den Pylorus (Magenausgang) 56 in den Magen gelangt, wenn dieser den Rückfluss nicht verhindern kann. Sie vermischt sich insbesondere in der Umgebung des Pylorus mit der Flüssigkeit, in der die Endoskopkapsel schwimmt und trübt sie ein. Diese lokale Trübung kann ebenfalls automatisch detektiert werden und erlaubt so Rückschlüsse auf die Funktionstüchtigkeit des Pylorus. Darüber hinaus kann auch aus der automatisch bestimmten farblichen Zusammensetzung der Flüssigkeit eine Leberfehlfunktion erkannt werden. In der Flüssigkeit auftretende Galle besteht aus verschiedenen chemischen Komponenten von denen einige klar sind und andere dagegen farbig sind, so dass die Farbe der Gallenflüssigkeit je nach Anteil der in der Galle befindlichen hauptsächlich Farbstoffe, nämlich gelbes Bilirubin und grünes Biliverdin von gelblich bis grünlich variiert. Beides sind Abbauprodukte der Leber von Hämoglobin.

Je nach Veränderung der Konzentrationen eines der beiden Abbauprodukte erscheint die Gallenflüssigkeit also mehr gelblich oder grünlich und lässt bei bestimmten Farbverschiebungen Rückschlüsse auf Leberfehlfunktionen zu. Dieser Farbunterschied bleibt erhalten bei der Vermischung mit der Flüssigkeitslösung im Arbeitsraum (Magen und/oder Darm) und kann anhand einer Analyse der Endoskopbilder mit Methoden der digitalen Bildverarbeitung oder -analyse automatisch detektiert werden.

## Patentansprüche

1. Einrichtung zur Untersuchung eines Hohlorgans mit einem Magnetsystem zum Führen einer mit einem Permanentmagneten versehenen Endoskopkapsel, die Videobilder aus dem Inneren des Hohlorgans aufnimmt und an eine Steuer- und Auswerteeinrichtung übermittelt, in der eine Software implementiert ist zum Durchführen während der Navigation der Endoskopkapsel (2) von Methoden der digitalen Bildverarbeitung so das die Videobilder automatisch auf das Vorhandensein diagnostisch relevanter Information analysiert werden, mit folgenden Merkmalen:
a) aus den Videobildern wird zum Lokalisieren von hohen Intensitätsgradienten durch Vorbearbeitung jeweils ein Grauwertbild erzeugt, dessen Bildpunkten jeweils ein Grauwert zugeordnet wird,
b) aus dem Grauwertbild wird durch Glättung und Kantenfilterung ein gefiltertes Bild erzeugt, dessen Bildpunkten ein Filterwert zugeordnet ist,
c) aus den Filterwerten der Bildpunkte des gefilterten Bildes wird ein Schwellwert abgeleitet,
d) das Videobild wird in eine Vielzahl von disjunkten Gruppen aufgeteilt, die jeweils eine Vielzahl von Bildpunkten umfassen,
e) aus diesen Gruppen werden als relevante Gruppen diejenigen Gruppen ausgewählt, die im gefilterten Bild eine Mindestanzahl von Bildpunkten enthalten, die den Schwellwert überschreiten,
f) jeder relevanten Gruppe wird eine Vielzahl von Eigenschaften zugeordnet, anhand derer mit einem mit einer Vielzahl von bewerteten Testbildern trainierten Expertensystem während der Navigation automatisch analysiert wird, ob die relevante Gruppe einen diagnostisch relevanten Bereich indiziert oder nicht,
g) das Ergebnis dieser Analyse wird zur Steuerung der Endoskopkapsel (2) herangezogen.

2. Einrichtung nach Anspruch 1, bei dem der Hohlorgan einen Magen ist.

3. Einrichtung nach Anspruch 1, bei dem die diagnostisch relevante Information in einer Trübung und/oder Färbung einer im Hohlorgan befindlichen Flüssigkeit und/oder einem Vorhandensein von Partikeln in der Flüssigkeit besteht.

4. Einrichtung nach einem der Ansprüche 1 bis 3, mit folgenden Merkmalen:
a) aus einem Videobild wird zum Lokalisieren von hohen Intensitätsgradienten durch Vorbearbeitung ein Grauwertbild erzeugt, dessen Bildpunkten ein Grauwert zugeordnet wird,
b) aus dem Grauwertbild wird durch Glättung und Kantenfilterung ein gefiltertes Bild erzeugt, dessen Bildpunkten ein Filterwert zugeordnet ist,
c) aus den Filterwerten der Bildpunkte des gefilterten Bildes wird ein Schwellwert abgeleitet,
d) das Videobild wird in eine Vielzahl von disjunkten Gruppen aufgeteilt, die jeweils eine Vielzahl von Bildpunkten umfassen,
e) aus diesen Gruppen werden als relevante Gruppen diejenigen Gruppen ausgewählt, die im gefilterten Bild eine Mindestanzahl von Bildpunkten enthalten, die den Schwellwert überschreiten,
f) die relevanten Gruppen können manuell als diagnostisch relevant oder diagnostisch irrelevant klassifiziert werden.

## Claims

1. Means for examining a hollow organ with a magnet system for guiding an endoscope capsule equipped with a permanent magnet, which records video images from the interior of the hollow organ and transmits these to a control and evaluation means, in which software for carrying out methods of digital image processing during the navigation of the endoscope capsule (2) is implemented, so that the video images are automatically analysed for the presence of diagnostically relevant information, with the following characteristics:
a) a grey tone image each is generated from the video images for localising high intensity gradients through pre-processing, the image points of which are each allocated a grey value,
b) a filtered image is generated from the grey value image through smoothing and edge filtering, the image points of which are allocated a filter value,
c) a threshold value is deducted from the filter values of the image points of the filtered image,
d) the video image is divided into a multitude of disjunctive groups, each comprising a multitude of image points,
e) those groups that contain a minimum number of image points that exceed the threshold value in the filtered image are selected from these groups as the relevant groups,
f) a multitude of characteristics is allocated to each relevant group, with the aid of which it can be automatically analysed during navigation with an expert system trained with a multitude of evaluated test images whether the relevant group indicates a diagnostically relevant area or not,
g) the result of this analysis is used for controlling the endoscope capsule (2).

2. Means according to claim 1, wherein the hollow organ is a stomach.

3. Means according to claim 1, wherein the diagnostically relevant information consists of a clouding and/or colouration of a fluid present in the hollow organ and/or a presence of particles in the fluid.

4. Means according to one of the claims 1 to 3, with the following characteristics:
a) a grey value image is generated from a video image for localising high intensity gradients through pre-processing, the image points of which are each allocated a grey value,
b) a filtered image is generated from the grey value image through smoothing and edge filtering, the image points of which are allocated a filter value,
c) a threshold value is deducted from the filter values of the image points of the filtered image,
d) the video image is divided into a multitude of disjunctive groups, each comprising a multitude of image points,
e) those groups that contain a minimum number of image points that exceed the threshold value in the filtered image are selected from these groups as the relevant groups,
f) the relevant groups can be manually classified as diagnostically relevant or diagnostically irrelevant.

## Revendications

1. Dispositif destiné à l'examen d'un organe creux avec un système magnétique afin de naviguer une capsule d'endoscopie munie d'un aimant permanent, ladite capsule enregistrant des images vidéo de l'intérieur de l'organe creux et les transmettant à un dispositif de commande et d'analyse dans lequel un logiciel est implémenté pour effectuer pendant la navigation de la capsule d'endoscopie (2) des méthodes du traitement numérique des images de sorte que les images vidéo soient analysées automatiquement quant à l'existence d'informations pertinentes pour le diagnostic, ledit dispositif présentant les caractéristiques suivantes :
a) une image en niveaux de gris dont les pixels sont respectivement attribués à un niveau de gris est créée à partir des images vidéo prétraitées pour la localisation de hauts degrés d'intensité,
b) une image filtrée dont les pixels sont attribués à une valeur filtre est créée à partir de l'image en niveaux de gris par lissage et filtrage des bords,
c) une valeur seuil est déduite à partir des valeurs filtres des pixels de l'image filtrée,
d) l'image vidéo est divisée en une multitude de groupes disjonctifs qui comprennent respectivement une multitude de pixels,
e) à partir de ces groupes, les groupes qui comprennent dans l'image filtrée un nombre minimum de pixels dépassant la valeur seuil sont sélectionnés comme des groupes pertinents,
f) chaque groupe pertinent est attribué à une multitude de propriétés à l'aide desquelles un système expert entraîné avec une multitude d'images d'essai évaluées analyse automatiquement pendant la navigation si le groupe pertinent indique ou non une zone pertinente pour le diagnostic,
g) le résultat de cette analyse est utilisé pour la commande de la capsule d'endoscopie (2).

2. Dispositif selon la revendication 1 selon laquelle l'organe creux est un estomac.

3. Dispositif selon la revendication 1 selon laquelle l'information pertinente pour le diagnostic est la turbidité et/ou la coloration d'un liquide situé dans l'organe creux et/ou l'existence de particules dans le liquide.

4. Dispositif selon l'une des revendications 1 à 3 présentant les caractéristiques suivantes :
a) une image en niveaux de gris dont les pixels sont attribués à un niveau de gris est créée à partir d'une image vidéo prétraitée pour la localisation de hauts degrés d'intensité,
b) une image filtrée dont les pixels sont attribués à une valeur filtre est créée à partir de l'image en niveaux de gris par lissage et filtrage des bords,
c) une valeur seuil est déduite à partir des valeurs filtres des pixels de l'image filtrée,
d) l'image vidéo est divisée en une multitude de groupes disjonctifs qui comprennent respectivement une multitude de pixels,
e) à partir de ces groupes, les groupes qui comprennent dans l'image filtrée un nombre minimum de pixels dépassant la valeur seuil sont sélectionnés comme des groupes pertinents,
f) les groupes pertinents peuvent être classés manuellement comme pertinents pour le diagnostic ou non pertinents pour le diagnostic.
